# EUROPEAN PATENT SPECIFICATION

(11) **EP 2 407 035 B1**
(45) Date of publication and mention of the grant of the patent: **22.02.2017**
(21) Application number: 10751072.9
(22) Date of filing: 03.03.2010
(51) Int. Cl.: A23P 10/28, A23L 33/10, A23L 33/105, A23L 33/15, A23L 33/16

(54) **BIOLOGICALLY ACTIVE FOOD SUPPLEMENT**
BIOLOGISCH AKTIVE NAHRUNGSERGÄNZUNG
ADDITIF BIOACTIF AUX ALIMENTS

(30) Priority: 13.03.2009 RU 2009109273
(43) Date of publication of application: 18.01.2012
(73) Proprietor: Obshestvo S Ogranichennoj Otvetstvennostju "PARAFARM", Penza 440023 (RU); Elistratov, Dmitriy Gennadjevich, Penza 440060 (RU)
(72) Inventor: ELISTRATOV, Dmitriy Gennadjevich, Penza 440060 (RU)
(74) Representative: Engel, Christoph Klaus
(86) International application number: PCT/RU2010/000098
(87) International publication number: WO 2010/104418

(56) References cited:
- DE-A1- 19 627 627
- KZ-A- 14 879
- RU-C1- 2 066 963
- RU-C1- 2 149 566
- RU-C1- 2 216 346
- RU-C1- 2 233 666
- SU-A3- 1 826 910
- DATABASE WPI Week 199720 Thomson Scientific, London, GB; AN 1997-224497 XP002687606, & RU 2 066 963 C1 (SEIFULLA R D) 27 September 1996 (1996-09-27)
- DATABASE WPI Week 200459 Thomson Scientific, London, GB; AN 2004-612541 XP002687607, & RU 2 233 666 C1 (PYATIGORSK PHARM ACAD) 10 August 2004 (2004-08-10)
- KHISMATULINA N.Z.: 'Apiterapiya' PERM vol. 77, 2005, pages 174 - 175, XP008167644

## Description

This invention refers to the food industry, in particular, to food supplements, intended to grow the athletes' muscle mass.

The best biostimulants and energy-rich products include the preparations developed based on apiculture products. There is a known preparation "Tonus" TU-64-02-06-244-91 developed based on flower pollen and lactose, and approved by the Chief Sanitary Department at the RF President, which is produced by BIOCOR. However, this preparation does not contain a multivitamin complex and vital microelements. Its effect upon the human body is governed only by the flower pollen, what may hamper the understanding of "Tonus" biological activity, as it is necessary to know precisely when the pollen is collected and from what plants.

RU 2 233 666 C1 shows a composition which is used as anabolic composition. The composition comprises male bee brood as active agent.

DE 196 27 627 A1 discloses a composition which comprises an extract of male bee brood. The composition may topically used for the treatment of muscle diseases.

The nearest to the invention by the technical substance and achieved result is the natural multivitamin complex "Leveton", Patent RU 2066963 C1 (IPC A23L001/076). It contains the flower pollen (pollen load), leuzea, ascorbic acid, α-tocopherol acetate, propolis, calcium stearate, talc and lactose with the following ratio of components (mass%): flower pollen (pollen load) 50-51, leuzea 10, ascorbic acid 3,0-3,5, α-tocopherol acetate 0.15, propolis 2, calcium stearate 0.92-0.95, talc 3, lactose - the remainder. "Leveton" is developed to increase the athletes' strength properties and muscle mass, and has been actively used by Schools of higher sportsmanship for over 10 years. The "Leveton" effect is conditioned by synergism of its components: precisely dosed vitamins and donators of phytoecdysteroids, namely, leuzea and flower pollen (pollen load).

In the proposed invention, the flower pollen (pollen load) is suggested to be substituted by male bee brood, a substance that possesses a stronger anabolic effect than flower pollen, serves as a natural drug enabling to increase the muscle mass, protect muscles from biochemical damages and achieve maximum results.

Male bee brood differs from the flower pollen by a larger number of functional groups of enzymes of sulphide groups, as well as hormones, namely, testosteroids, progesterone and estradiol. Thanks to this set of substances, male bee brood promotes an increased restoration of biochemical and mass-metric characteristics of spermary and prostate gland, being a stimulant of central regulatory mechanisms of androgen formation intensity.

Chemical composition: proteins 10-20%; carbohydrates 1-5.5%; fats 5-6.3%; amine acids 11.4%; glucose 3.18-5%; fructose and saccharose up to 0.5%.

Microelements (mg%): K 0.50, Na 38, Ca 14, P 189, Mg 2, Fe 3.23, Mn 4.40%, Zn 5.54, Cu 2, Cr, Co, Ni, Ag, Au, and others.

Vitamins (water- and liposoluble): A 0.54 IU/g; xanthophyll 0.297mg%; B-carotine 0.426 IU/g; B2 0.739 mg%; D 950 IU/g; choline 442.8 mg%; nicotinic acid 15.8 mg%.

The male bee brood increases the level of metabolism during the period of active muscle activity owing to which physical endurance raises. The male bee brood contains 10 times more steroid hormones compared to the flower pollen.

As of today, "Leveton" does not meet current requirements to rapid growth of the athletes' muscle mass, does not ensure rapid recovery of fatigued muscles after a hard physical load. The proposed invention makes the process of an athlete's recovery shorter compared to a prototype at the expense of a higher content of phytoecdysteroids.

The above technical result is achieved owing to the fact that the male bee brood is introduced additionally into the known therapeutic-prophylactic product "Leveton" containing leuzea, ascorbic acid (Vitamin C) and Vitamin E and excipients, and the pollen load is removed from its composition with the following ratio of components (mass%):
Male bee brood 1-70;
Leuzea 5-70;
Ascorbic acid 1-70;
Vitamin E 0.05-30;
Excipients- the rest.

As excipients, one may use for instance, calcium stearate 0.92-0.95, talc 3-6, lactose - the remainder.

In the process of search by sources of scientific-technical and patent information, no substance was found to possess a similar aggregate of essential features with the same positive effect achieved. Thus, the proposed invention presents a technical solution of the task, possesses a novelty, an inventive level and industrial usefulness. The therapeutic-prophylactic product is manufactured in a tablet form. A low level is determined by convenience of use of the proposed complex, i.e., the use of smaller number of tablets. An upper level is determined by the fact that in case of greater percent ratio, it will be difficult to manufacture a tablet as it will fall to pieces.

### Example 1

An example of the mix formulation for tablet mass 500g:

| | |
|---|---|
| Male bee brood | 10 mg |
| Leuzea | 50 mg |
| Ascorbic acid | 50 mg |
| Vitamin E | 5 mg |
| Excipients | 385 mg |

Thus, the proposed food supplement developed based on leuzea, male bee brood, ascorbic acid (Vitamin C), Vitamin E, excipients may be used as a product for the athletes' muscle mass growth owing to an increased content of phytoecdysteroids, phytohormones influencing the anabolic processes in an athlete's body.

The product not only renders an anabolic effect on the human body, but is recommended for prophylaxis of dysfunctions of the central nervous system, treatment of diseases of the prostate gland and the loss of sexual power as well.

## Claims

1. A food supplement, containing leuzea, ascorbic acid (Vitamin C) and Vitamin E and excipients (calcium stearate, talc, lactose), differing in that it contains a male bee brood with the following ratio of components (mass%) :
Male bee brood 1-70;
Leuzea 5-70;
Ascorbic acid 1-70;
Vitamin E 0.05-30;
Excipients- the rest.

## Patentansprüche

1. Nahrungsergänzungsmittel, enthaltend Leuzea, Ascorbinsäure (Vitamin C) und Vitamin E sowie Trägerstoffe (Calciumstearat, Talk, Laktose), **dadurch gekennzeichnet, dass** es Drohnenbrut mit dem folgenden Verhältnis der Bestandteile enthält (in Masse %):
- Drohnenbrut 1-70;
- Leuzea 5-70;
- Ascorbinsäure 1-70;
- Vitamin E 0,05-30;
- Trägerstoffe - der Rest.

## Revendications

1. Supplément alimentaire, contenant de la leuzée, de l'acide ascorbique (vitamine C) et de la vitamine E et des excipients (stéarate de calcium, talc, lactose), différant en ce qu'il contient un couvain de faux bourdon avec les taux suivants de composants (% en poids) :
couvain de faux bourdon 1 à 70 ;
leuzée 5 à 70 ;
acide ascorbique 1 à 70 ;
vitamine E 0,05 à 30 ;
excipients - le reste.
